# EUROPEAN PATENT APPLICATION

(11) **EP 1 813 263 A1**
(43) Date of publication of application: **01.08.2007**
(21) Application number: 06001682.1
(22) Date of filing: 27.01.2006
(51) Int. Cl.: A61K 8/81, A61Q 5/10

(54) **Agent for the nonoxidative coloring of hair**

(71) Applicant: Wella Aktiengesellschaft, 64274 Darmstadt (DE)
(72) Inventor: Aeby, Johann, 1723 Marly (CH); Pauli, Irène, 1792 Guschelmuth (CH); Mürner, Hans Rudolf, 1717 St. Ursen (CH)
(74) Representative: Bockhorst, Matthias

(57) **Abstract**

The present invention relates to nonoxidative hair dyes based on acidic dyes that contain Polyacrylate-13 as a thickener.

## Description

The present invention relates to a gel-type or creme-type nonoxidative hair dye based on acidic dyes.

For the coloring of hair, in addition to oxidative hair dyes that are formed through the oxidative coupling of one or more developer components, direct-penetrating dyes are gaining an increasing level of importance. Of particularly increasing importance in this regard are hair dyes based on acidic dyes, which enable an especially gentle hair coloring.

However, a problem that arises with such hair dyes is the frequent occurrence of strong coloration of the scalp as well as of the neighboring areas of skin. A possibility for avoiding this problem involves the use of a gel-type hair dye preparation, in which said gel is applied to the hair precisely at a distance of 1 mm to 2 mm away from the scalp while avoiding any skin contact. However, this is not possible with currently known hair dye gels, which employ cellulose for thickening, since these gels exhibit inadequate adhesion to hair and run off the hair relatively easily.

Attempts have been made to eliminate this problem through the use of other thickeners. Thus, it is known from JP-A 2000-159644 and JP-A 2000-128748 that certain acrylamide homopolymers can be used for the thickening of acidic hair dyes. Likewise, it is known from WO 01/35922 that certain acrylamide copolymers can be used in the thickening of hair treatment agents. Furthermore, JP-A 08-245348 describes the use of crosslinked polyacrylates for the thickening of acidic hair dyes. However, the properties of these agents based on polyacrylamides or polyacrylic acid are not satisfactory in all respects.

There is thus a significant need for a hair dye gel based on acidic dyes that is easy to manufacture and is stable, and at the same time enables a uniform coloring of the hair without any coloration of the scalp.

It has now been found that the use of certain acrylate copolymers singly or in combination with certain polysorbates fulfill to a great extent the aforementioned requirements for the thickening of hair dyes.

The object of the present invention is thus an agent for the coloring of hair that is based on at least one acidic dye, wherein said agent contains a copolymer of acrylic acid and 2-methyl-2-[(1-oxo-2-propenyl)amino]-1-propanesulfonic acid monosodium salt, 2-propenamide and sodium 2-propenoate (Polyacrylate-13; CAS-Nr.: 15728-72-8) as a thickener. ,

The hair dye of the present invention contains Polyacrylate-13 preferably in an amount of from about 0.01 to 1 percent by weight, especially in an amount of from 0.1 to 0.5 percent by weight.

In addition to Polyacrylate-13, the colorant of the present invention preferably contains at least one isoalkane polymer, especially polyisobutene, and/or at least one nonionic surfactant based on ethoxylated sorbitan laurate esters, especially with an average degree of ethoxylation of 20 (for example, Polysorbate-20). An especially preferred combination of the aforementioned three compounds of the present invention, for example, is distributed under the trade name SEPIPLUS 400 by the Seppic Company.

The amount of isoalkane polymer used in the colorant of the present invention is preferably from about 0.06 to 2 percent by weight, while the nonionic surfactant is preferably used in an amount of from about 1.5 to 3.2 percent by weight.

Furthermore, the hair dye of the present invention contains at least one acidic dye or a mixture of several acidic dyes, wherein the total quantity of these dyes preferably amounts to from 0.01 to 5 percent by weight.

Known acidic food dyes can be used as the acidic dye, as well as the acidic dyes named in the CTFA International Cosmetic Ingredient Dictionary, 10th Edition (2004) such as "D&C dyes," "Ext. D&C dyes" and "FD&C dyes" as well as other "Acid Dyes" described therein. The following examples of suitable acidic dyes can be named in particular: D&C Yellow No. 8 (C.I. 45350), D&C Red No. 31 (C.I. 15800), D&C Red No. 22 (C.I. 45380), D&C Orange No. 4 (C.I. 15510), D&C Green No. 5, D&C Red No. 6 (C.I. 15850), FD&C Green No. 3 (C.I. 42053), FD&C Blue No. 1 (C.I. 42090), Ext. D&C Violet No. 2 (C.I. 60730), Ext. D&C Yellow No. 7 (C.I. 10316), Acid Black 1 (C.I. 20470), Acid Blue 3 (C.I. 42051), Acid Green 25 (C.I. 61570), Acid Green 1 (C.I. 10020), Acid Orange 3 (C.I. 10358), Acid Orange 24 (C.I. 20170), Acid Red 27 (C.I. 16185), Acid Red 18 (C.I. 16255), Acid Red 52 (C.I. 45100), Acid Violet 9 (C.I. 45190), Acid Yellow 1 (C.I. 10316) and Acid Yellow 23 (C.I. 19140).

Especially preferred dyes are Acid Red 52, Acid Red 18, D&C Red No. 6, Acid Yellow 1, Acid Black 1, Acid Orange 7 (D&C Orange No. 4) and Acid Violet 43 (Ext. D&C Violet No. 2).

The named dyes are characterized by the fact that they can be used for hair coloring in an acidic medium, in order to intensify the coloring of the hair. The colorants of the present invention thus possess an acidic pH value of from 1.5 to 7, whereby the desired pH value can generally be adjusted with organic acids, such as for example lactic acid, glycolic acid, succinic acid, malic acid, tartaric acid or inorganic acids, for example phosphoric acid.

In order to improve the coloring performance, additional carrier substances, such as for example benzyl alcohol, N-methylpyrrolidone, 1,3-butanediol, phenylethyl alcohol, propylene glycol and n-propanol can be added, or other solvents that promote the penetration of the dyes can be added to colorants of the present invention. The addition of benzyl alcohol and/or propylene glycol is preferred in this regard, wherein the total quantity of benzyl alcohol and propylene glycol is from about 2 to 25 percent by weight. Furthermore, triethylene glycol monobutyl ether and 2-benzyloxyethanol as well as ethanol and isopropanol can be added. The total quantity of ethanol and isopropanol amounts to from about 2 to 25 percent by weight.

In an especially preferred embodiment, the agent of the present invention additionally contains a polyglucoside, for example coco glucoside (Plantacare® 818 UP of the Cognis/BRD Company). The amount of the polyglucoside used is preferably from about 0.1 to 3 percent by weight, especially from 0.5 to 2 percent by weight.

The hair dye of the present invention can further contain all conventional and known additives for such preparations, for example perfume oils; chelating agents; cosmetic resins, such as for example polyvinylpyrrolidone or poly(vinylacetate); alginates; guar gum; hair-conditioning substances, such as for example cationic polymers or lanolin derivatives; or wetting agents and emulsifiers from the classes of anionic, nonionic, amphoteric or cationic surface-active substances.

The components mentioned are used in the usual amounts for such purposes, for example the wetting agents and emulsifiers are used in a concentration of from 0.1 to 30 percent by weight and the conditioning agents are used in amounts of from 0.1 to 5 percent by weight.

The hair dye of the present invention exhibits a relatively high water content of from about 25 to 90 percent by weight, preferably from 30 to 70 percent by weight.

The use of a hair dye of the present invention follows known procedures, in which one takes an amount of the hair coloring that is sufficient for the length of the hair, from about 30 g to 120 g, the hair dye is then applied to the hair, the hair dye is allowed to act at a temperature of from 15 °C to 50 °C for a period of from about 5 to 50 minutes, after which the hair is thoroughly rinsed with water and dried.

The hair dye of the present invention does not run off the hair and has very good appliability, so that excellent coloring of the hair can be achieved without the simultaneous coloration of the scalp.

A further advantage of the hair dye of the present invention is the easy producibility, whereby practically no problems with dust arise during production, as well as the high stability of the agent, especially with respect to the agent undergoing separation.

The following examples should further illustrate the object of the present invention, while the invention is not limited to these examples.

### Examples

### Example 1: Hair dye

| | |
|---|---|
| 0.1 g | D&C Red No. 6 (C.I. 15850) |
| 4.0 g | SEPIPLUS 400 from the Seppic Company (mixture of 60% Polysorbate-20, 30% polyisobutene, 5% Polyacrylate-13 and 5% water) |
| 9.0 g | benzyl alcohol |
| 1.0 g | coco glucoside (Plantacare^{®} 818UP from the Cognis Company; 53% aqueous solution) |
| 0.5 g | hydroxyethylcellulose (Natrosol^{®} 250 HHX) |
| 0.3 g | perfume |
| 10.0 g | 1,2-propylene glycol |
| 5.0 g | ethanol |
| balance to 100.0 g | water |

50 g of the above-mentioned hair dye was applied to hair that has previously been washed and rubbed down, while care was taken that none of the coloring gel reached the scalp and the neighboring areas of skin. After an action period of 20 minutes at room temperature (25 °C), the hair was rinsed with water and then dried.
Hair treated in this manner possesses an intense red color.

### Example 2: Hair dye

| | |
|---|---|
| 0.1 g | Acid Black 1 (C.I. 20 470) |
| 4.0 g | SEPIPLUS 400 from the Seppic Company (mixture of 60% Polysorbate-20, 30% polyisobutene, 5% Polyacrylate-13 and 5% water) |
| 9.0 g | benzyl alcohol |
| 1.0 g | coco glucoside (Plantacare^{®} 818UP from the Cognis Company; 53% aqueous solution) |
| 0.5 g | hydroxyethylcellulose (Natrosol^{®} 250 HHX) |
| 0.3 g | perfume |
| 10.0 g | 1,2-propylene glycol |
| 5.0 g | ethanol |
| balance to 100.0 g | water |

50 g of the above-mentioned hair dye was applied to hair that has previously been washed and rubbed down, while care was taken that none of the coloring gel reached the scalp and the neighboring areas of skin. After an action period of 15 minutes at 40 °C, the hair was rinsed with water and then dried.
Hair treated in this manner possesses a deep dark black color.

### Example 3: Hair dye

| | |
|---|---|
| 0.19 g | D&C Orange No. 4 (C.I. 15 510) |
| 0.03 g | Black 401 (C.I. 20 470) |
| 0.06 g | Ext. D&C Violet No. 2 (C.I. 60730) |
| 4.00 g | SEPIPLUS 400 from the Seppic Company (mixture of 60 Polysorbate-20, 30% polyisobutene, 5% Polyacrylate-13 and 5% water) |
| 6.00 g | benzyl alcohol |
| 1.00 g | coco glucoside (Plantacare^{®} 818UP from the Cognis Company; 53% aqueous solution) |
| 1.00 g | hydroxyethylcellulose (Natrosol^{®} 250 HHX) |
| 0.30 g | perfume |
| 10.00 g | 1,2-propylene glycol |
| 5.00 g | ethanol |
| balance to 100.00 g | water |

50 g of the above-mentioned hair dye was applied to hair that has previously been washed and rubbed down, while care was taken that none of the coloring gel reaches the scalp and the neighboring areas of skin. After an action period of 20 minutes at room temperature (25 °C), the hair was rinsed with water and then dried.
Hair treated in this manner possesses a brown color.

### Example 4: Hair dye

| | |
|---|---|
| 0.10 g | Red 227 (C.I. 17 200) |
| 0.08 g | Red 201 (C.I. 15 850) |
| 4.00 g | SEPIPLUS 400 from the Seppic Company (mixture of 60% Polysorbate-20, 30% polyisobutene, 5% Polyacrylate-13 and 5% water) |
| 9.00 g | benzyl alcohol |
| 0.50 g | coco glucoside (Plantacare^{®} 818UP from the Cognis Company; 53% aqueous solution) |
| 0.50 g | hydroxyethylcellulose (Natrosol^{®} 250 HHX) |
| 1.00 g | betaine |
| 0.30 g | perfume |
| 5.00 g | 1,2-propylene glycol |
| 10.00 g | ethanol |
| balance to 100.00 g | water |

50 g of the above-mentioned hair dye was applied to hair that has previously been washed and rubbed down, while care was taken that none of the coloring gel reaches the scalp and the neighboring areas of skin. After an action period of 15 minutes at 40 °C, the hair was rinsed with water and then dried.
Hair treated in this manner will possess an intense red color.
Unless indicated otherwise, all percent values represent percent by weight.

## Claims

1. An agent for the coloring of hair that is based upon at least one acidic dye, **characterized in that** said agent contains Polyacrylate-13 as a thickener.

2. The agent according to Claim 1, **characterized in that** Polyacrylate-13 is contained in an amount of from 0.01 to 1 percent by weight.

3. The agent according to Claim 1 or 2, **characterized in that** the acidic dye is selected from among Acid Red 52, Acid Red 18, D&C Red No. 6, Acid Yellow 1, Acid Black 1, Acid Orange 7 and Acid Violet 43.

4. The agent according to one of Claims 1 to 3, **characterized in that** the total quantity of acidic dye contained is from 0.01 to 5 percent by weight.

5. The agent according to one of Claims 1 to 4, wherein said agent contains from 2 to 25 percent by weight of benzyl alcohol and/or propylene glycol.

6. The agent according to one of Claims 1 to 5, **characterized in that** said agent additionally contains at least one isoalkane polymer and/or at least one nonionic surfactant.

7. The agent according to Claim 6, **characterized in that** the isoalkane polymer is polyisobutene.

8. The agent according to Claim 6, **characterized in that** the nonionic surfactant is Polysorbate-20.

9. The agent according to Claim 6 or 7, **characterized in that** the isoalkane polymer is contained in an amount of from 0.06 to 2 percent by weight.

10. The agent according to Claim 6 or 8, **characterized in that** the nonionic surfactant is contained in an amount of from 1.5 to 3.2 percent by weight.

11. An agent for the coloring of hair, **characterized in that** said agent contains
(a) from 0.01 to 5 percent by weight of at least one acidic dye;
(b) from 0.01 to 1 percent by weight of Polyacrylate-13;
(c) from 0.06 to 2 percent by weight of polyisobutene; and,
(d) from 1.5 to 3.2 percent by weight of Polysorbate-20.

12. The agent according to one of Claims 1 to 11, **characterized in that** said agent contains from 25 to 90 percent by weight of water.

13. The agent according to one of Claims 1 to 12, **characterized in that** said agent additionally contains at least one polyglucoside.

14. The agent according to Claim 13, **characterized in that** the polyglucoside is contained in an amount of from 0.1 to 3 percent by weight.

15. The agent according to one of Claims 1 to 14, **characterized in that** said agent exhibits a pH value of from 1.5 to 7.
